# EUROPEAN PATENT SPECIFICATION

(11) **EP 3 802 865 B1**
(45) Date of publication and mention of the grant of the patent: **10.04.2024**
(21) Application number: 19733871.8
(22) Date of filing: 22.05.2019
(51) Int. Cl.: C12Q 1/6816, C12Q 1/682, C12Q 1/6834

(54) **KIT AND METHOD FOR THE COLORIMETRIC DETECTION OF A TARGET NUCLEIC ACID IN A SAMPLE**
KIT UND VERFAHREN ZUM KOLORIMETRISCHEN NACHWEIS EINER ZIELNUKLEINSÄURE IN EINER PROBE
KIT ET PROCÉDÉ DE DÉTECTION COLORIMÉTRIQUE D'UN ACIDE NUCLÉIQUE CIBLE DANS UN ÉCHANTILLON

(30) Priority: 24.05.2018 IT 201800005685
(43) Date of publication of application: 14.04.2021
(73) Proprietor: Fondazione Istituto Italiano di Tecnologia, 16163 Genova (IT)
(72) Inventor: TATULLI, Giuseppina, 70038 Terlizzi (Bari) (IT); VALENTINI, Paola, 16131 Genova (IT); POMPA, Pier Paolo, 16146 Genova (IT)
(74) Representative: Comoglio, Elena
(86) International application number: PCT/IB2019/054215
(87) International publication number: WO 2019/224731

(56) References cited:
- EP-A1- 1 400 601
- WO-A1-2011/090445
- US-A1- 2006 063 925
- US-A1- 2010 000 881
- US-A1- 2010 129 819
- MA M ET AL: "Peroxidase-like catalytic activity of cubic Pt nanocrystals", COLLOIDS AND SURFACES A: PHYSIOCHEMICAL AND ENGINEERING ASPECTS, ELSEVIER, AMSTERDAM, NL, vol. 373, no. 1-3, 3 January 2011 (2011-01-03), pages 6-10, XP027552780, ISSN: 0927-7757 [retrieved on 2010-08-12]

## Description

The present invention falls within the field of molecular biology.

More particularly, the present invention relates to a method and a kit for the colorimetric detection of a target nucleic acid in a sample of interest in which the target nucleic acid is present in mixture with other sample materials, including other nucleic acids different from the target nucleic acid in the sequence of bases.

In *in vitro* diagnostics, the detection of nucleic acids (dsDNA, ssDNA or RNA) is of fundamental importance for a wide range of applications, from clinical diagnostics to quality control and academic research.

However, in almost all applications on real samples, the target nucleic acid concentrations are extremely low, in the femtomolar range or lower, since the target nucleic acid is typically diluted in a mixture of interfering material, so it is practically impossible to detect it directly without first amplifying it.

In standard laboratory tests, the amplification of nucleic acids is generally carried out by means of PCR reactions or variants thereof, such as real-time PCR or digital PCR. PCR-based techniques are extremely versatile, specific and sensitive, but require dedicated instrumentation and precise thermal cycles in order to amplify the genetic material and therefore detect the target. These features have led to the fact that PCR is currently the gold-standard technique for laboratory analysis. However, this technique is difficult to apply for point-of-care analyses, which on the contrary would require extremely simple, portable, rapid, low-cost measurement systems usable by non-specialized personnel.

In this context, research focused on the development of simplified PCR-coupled techniques for detecting nucleic acids, such as for example colorimetric detection based on gold nanoparticles (in this regard, see for example international patent application WO 2016/097953) or on the development of novel nucleic acid isothermal detection techniques. The use of isothermal reactions could in fact give a significant impulse to the development of diagnostic tests for which simplified instrumentation is sufficient, which can be used outside specialized laboratories. A thorough review of the main nucleic acid isothermal detection techniques is Y. Zhao et al., "Isothermal Amplification of Nucleic Acids" Chem Rev, 2015, 115, 12491-12545.

However, many of these isothermal techniques have technical drawbacks that so far have limited the diffusion and actual extensive application thereof in commercial molecular assays. Some examples of these drawbacks are the need to use complex enzyme and/or protein mixtures, with a consequent increase in costs; the need for post-processing operations, and limited versatility; non-specific amplification problems, which are typical of techniques based on the combination of a polymerase and an endonuclease capable of single-strand cutting ("nicking"); the need to design complex amplification primer mixtures, with a consequent increase in costs.

Many of the isothermal techniques known in the state of the art, based on a preliminary enzymatic amplification step, are also characterised by multiple joint problems. Generally, most of these techniques have serious specificity and sensitivity issues, so, apart from a few specific applications, the vast majority of commercially available molecular assays are still based on the PCR.

US 2010/0000881 discloses a nucleic acid hybridization detection assay carried out at an electrode which is modified by capture probes comprising single-stranded oligonucleotides. In an embodiment using sandwich assay methodology, the capture probes hybridize to complementary target nucleic acid sequences, which in turn hybridize to detection probes comprising nanoparticle-oligonucleotide conjugates comprising target-complementary oligonucleotides. When the assay is carried out in the presence of a redox mediator, such as, *inter alia,* 3,3',5,5'-tetramethylbenzidine (TMB), redox reactions catalysed by the presence of nanoparticles generate electrons that are transferred to the electrode, resulting in a detectable electrical signal. The assay disclosed in US 2010/0000881 has a detection limit of about 10 µM.

In order to achieve PCR sensitivity, several hybrid approaches have also been attempted, by combining molecular biology techniques and nanomaterials, for example exploiting the plasmonic properties of the latter to increase the sensitivity of the assay. Techniques that exploit target amplification strategies, signal amplification strategies, or ultra-sensitive instrumental techniques (such as, for example, surface-enhanced Raman spectroscopy, atomic force microscopy, mass spectroscopy) are described in the literature, but none of these innovative technologies have actually evolved, to date, into a widely diffused or commercially relevant assay. For example, the use of enzymes (or enzyme mixtures) in isothermal signal or target amplification techniques typically has major disadvantages, such as high costs and limited thermal stability.

On the other hand, no rapid, simple and portable methods for detecting nucleic acids (DNA and RNA) are known, which do not require prior amplification of the target and which allow clinically relevant nucleic acid concentrations, for example femtomolar concentrations or lower, to be detected.

In some recent publications the attempt to exploit the properties of nanomaterials to create new high sensitivity diagnostic assays is described. For example, gold/silver nanoparticles are extremely efficient colorimetric tags due to the high molar extinction coefficients, far superior to the classic organic dyes. In this regard, see international patent applications WO 2016/097953 and WO 2016/097952. However, these properties are not yet sufficient to obtain a direct detection of DNA or RNA when its concentration in the test sample is in the sub-picomolar range. It was then attempted to increase the sensitivity of these nanoparticle tags through silver/gold/copper enhancement plans, which exploit the *in situ* metal deposition and the consequent growth of nanoparticles (D. Kim et al., "Microarray-Based Multiplexed Scanometric Immunoassay for Protein Cancer Markers Using Gold Nanoparticle Probes" Analytical Chemistry, 2009, 81, 9183-9187; X. Wei et al., "A selective resonance scattering assay for immunoglobulin G using Cu(II)-ascorbic acid-immunonanogold reaction" Anal Biochem, 2008, 380, 223-228).

Another strategy described in the literature is based on the excellent catalytic activity of some nanoparticles due to their high surface/volume ratio (Z. Wang et al., "Label-free detection of DNA by combining gated mesoporous silica and catalytic signal amplification of platinum nanoparticles" Analyst, 2014, 139, 6088-6091; Collins & Holmes, J. D. Engineering Metallic Nanoparticles for Enhancing and Probing Catalytic Reactions. Advanced Materials, 2016, 28, 5689-5695). To date, however, no methods have yet been described that combine specific and sensitive detection with low cost and portability.

One object of the present invention is thus to overcome the above shortcomings of the state of the art.

Another object of the present invention is to provide a rapid, portable and universal method which allows a target nucleic acid to be detected in a sample in which it is present in an extremely low concentration, for example a femtomolar or lower concentration, without the need for particular instrumentation or prior amplification steps, which would greatly facilitate the diagnostic and clinical use thereof.

These and other objects are achieved by the method and the related kit for the colorimetric detection of a target nucleic acid in a sample, as defined in independent claims 1 and 8, respectively.

Further features and advantages of the invention are defined in the dependent claims.

The claims form an integral part of the present specification.

The method and the kit of the invention use metal nanoparticles that are characterised by a peroxidase-like catalytic activity; preferably, these are platinum and/or palladium nanoparticles having a diameter of from about 1 nm to about 200 nm, preferably from about 5 to about 30 nm, still more preferably from about 8 to about 22 nm. Specific non-limiting examples are metal nanoparticles (NP) with a diameter of 8, 9, 10, 11, 12, 13, 14, 15, 16, 17, 18, 19, 20, 21 or 22 nm. The aforesaid metal nanoparticles are functionalized with probe molecules designated as universal oligonucleotide probes which, upon recognition of the target sequence present in the sample by two target-specific oligonucleotide probes, are captured onto a solid substrate and, in the presence of a chromogenic peroxidase substrate, generate a colour that can be detected with the naked eye even when the target nucleic acid is present in the sample in extremely low concentrations.

Therefore, the detection method of the present invention advantageously features a very high sensitivity - in the femtomolar range, for example 100 fM or even lower - a remarkable rate (the run time of the test is typically between a few seconds and 20 minutes), an isothermal character (the running temperature is typically between room temperature and 60°C), and does not require the use of *ad hoc* instrumentation (detection is carried out with the naked eye) or prior target amplification steps.

The method of the invention is superior to any current technology for nucleic acid detection based on the use of enzymes, both in terms of costs and in terms of robustness and stability to environmental conditions.

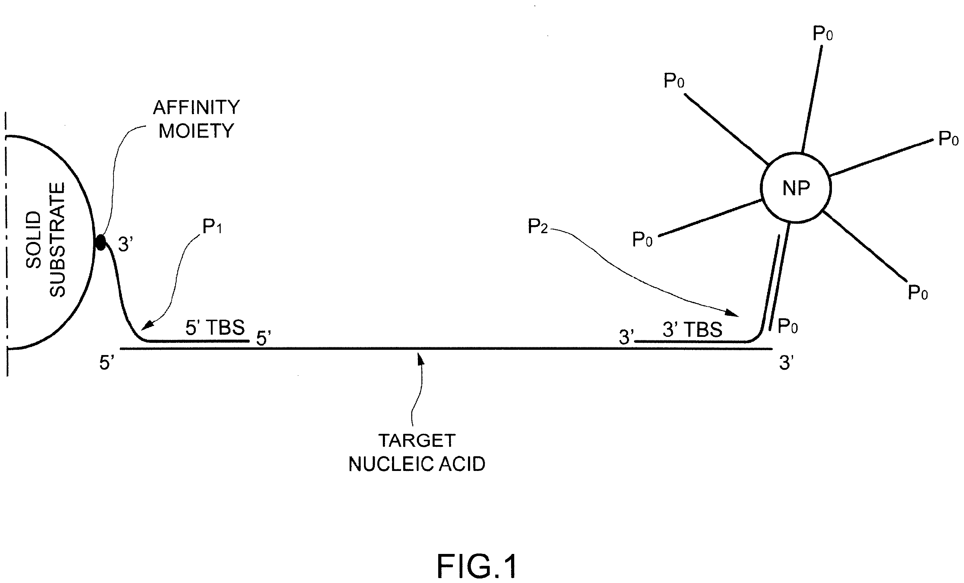

The method of the present invention, and the related kit, are described in greater detail below, with reference to Figure 1 which shows a diagram of the mechanism of operation of the method.

Figure 1 shows the metal nanoparticles characteristic of the present invention, referred to as NP in the figure, which possess a peroxidase-like catalytic activity and are functionalized with a universal oligonucleotide probe, referred to as P0 in the figure. The metal nanoparticles (NP) are preferably platinum and/or palladium nanoparticles and preferably have a diameter comprised between 1 nm and 200 nm, more preferably between 5 and 30 nm, still more preferably between 8 and 22 nm. Specific non-limiting examples are metal nanoparticles with a diameter of 8, 9, 10, 11, 12, 13, 14, 15, 16, 17, 18, 19, 20, 21 or 22 nm. The examples of the present specification relate to nanoparticles with a diameter of 10 and 20 nm.

The universal oligonucleotide probe P0 is a nucleic acid molecule that has a non-specific base sequence, i.e. not characteristic of the target nucleic acid. An example of such a universal oligonucleotide probe P0 is an oligonucleotide consisting of or comprising a polyT tail or a polyA tail. The length of the probe P0 is preferably comprised between 10 and 70 nucleotides, more preferably between 20 and 50 nucleotides; specific non-limiting examples are 25, 30, 35, 40, 45 nucleotides.

Figure 1 also shows two target-specific oligonucleotide probes referred to as first oligonucleotide probe P1 and second oligonucleotide probe P2, respectively, used in the method of the invention.

P1 probe comprises a 5' target binding sequence, referred to as 5'TBS in the figure, which is capable of hybridizing to a 5' region of the target nucleic acid. P1 probe further comprises a 3' sequence which is not capable of hybridizing to the target nucleic acid and is labelled at its 3' end with an affinity moiety, preferably biotin.

P2 probe comprises a 3' target binding sequence, referred to as 3'TBS in the figure, which is capable of hybridizing to a 3' region of the target nucleic acid. P2 probe further comprises a 5' sequence which is not capable of hybridizing to the target nucleic acid, but capable of hybridizing to the universal oligonucleotide probe P0 with which the metal nanoparticles are functionalized.

The length of the P1 and P2 probes is preferably comprised between 10 and 70 nucleotides, more preferably between 13 and 40 nucleotides for the P1 probe and between 33 and 60 nucleotides for the P2 probe. Specific non-limiting examples for the P1 probe are 15, 20, 25, 30, 35 nucleotides. Specific non-limiting examples for the P2 probe are 35, 40, 45, 50, 55 nucleotides.

Lastly, Figure 1 shows a solid substrate, for example magnetic beads, which is functionalized with the binding partner of the affinity moiety linked to the 3' end of the P1 probe. Binding partners for biotin are avidin and streptavidin.

In the presence of target nucleic acid (RNA, ssDNA or previously denatured dsDNA), P1 probe hybridizes to a 5' region of the target strand with its own 5'TBS sequence and P2 probe hybridizes to a 3' region of the target strand with its own 3'TBS sequence. Hybridization occurs on the basis of the principle of complementary base pairing. Since the solid substrate is functionalized with the binding partner of the affinity moiety with which the 3' end of the P1 probe is labelled, and since the NP metal nanoparticles are functionalized with the universal probe P0 which hybridizes to the 5' sequence of the P2 probe, in the presence of the target the sandwich structure, shown in Figure 1, is bound to the solid substrate. All the sample material not binding to the solid substrate is removed by washing and then the chromogenic peroxidase substrate is added. An exemplary chromogenic peroxidase substrate suitable for use in the method of the present invention is 3,3',5,5'-tetramethylbenzidine (TMB). Other chromogenic substrates as alternatives to TMB are 2,2'-azino-bis(3-ethylbenzothiazoline-6-sulfonic) acid (ABTS), 2,2-diphenyl-1-picrylhydrazyl (DPPH) or o-phenyldiamine (ODP).

In the presence of the target, the NP metal nanoparticles bound to the solid substrate perform their activity as oxidation catalysts and favour the development of a colour, which can be detected with the naked eye. In the absence of the target, instead, the NP metal nanoparticles do not bind to the solid substrate and are washed away, so that the addition of the chromogenic substrate does not result in colour development.

As indicated above, the target sequence can be DNA, for example genomic DNA (viruses, bacteria or other pathogens) or a barcoding sequence (for food traceability applications), or RNA.

If the target is dsDNA, the first step in the detection method of the invention is the thermal denaturation of the target DNA molecule. Alternatively, if the target is single-stranded (for example in the case of miRNA), denaturation is not performed and the step of hybridization of the P1 and P2 probes to the target is carried out directly. In both cases, after the hybridization step (which is generally carried out at a constant temperature, from RT to 60°C) the target is captured onto the surface of the solid substrate through biotin-streptavidin interaction (or similar interactions) and subsequently all the material not bound to the solid substrate is washed away.

According to one embodiment of the invention, the P2 probe is preliminarily hybridized to the P0 probe with which the NP metal nanoparticles are functionalized. In another alternative embodiment, the hybridization of the P2 probe to the P0 probe of the NP metal nanoparticles is carried out in a second step, after washing.

Similarly, according to one embodiment of the invention, the capture of the P1 probe onto the solid substrate takes place directly during the step of hybridization of the P1 and P2 probes to the target. In another alternative embodiment, instead, the capture of the P1 probe onto the solid substrate occurs immediately after the hybridization of the P1 and P2 probes to the target.

Generally, the time sequence of the various steps can be modified in different ways, including the execution of the reaction in one step (with the exception of the optional denaturation step which, if present, must be carried out beforehand), provided that the final result guarantees specific target capture by the solid substrate.

Thanks to the very high catalytic activity of the NP metal nanoparticles, the target is colorimetrically detected with the naked eye very quickly (typically 5-10 minutes, but if the target concentration is not low, the minimum detection time is reduced to around 30-60 seconds). Since the detection step is typically completed in about 5-10 minutes, the entire assay lasts about 20 minutes or even less. In an automated system, the entire assay can be expected to last about 10 minutes.

Potential specific applications of the method and the kit of the present invention are listed below:
- detection of any DNA or RNA sequence, without target amplification steps (for example, detection of pathogens);
- detection of a barcoding sequence for genetic authentication of food (isothermal, PCR-free process);
- PCR-free detection of a DNA point mutation by exploiting cooperative hybridization on nanoparticles;
- detection of a non-nucleic acid target (for example an analyte detectable by an aptamer), in a configuration in which one of the two probes is an aptamer.

The following examples are provided for illustration purposes only and do not limit the scope of the invention as defined in the appended claims.

### Example

The inventors carried out proof-of-principle tests to demonstrate that the entire detection method plan according to the invention works correctly, from the functionalization of platinum nanoparticles (NP-Pt) with DNA probes, to the specific recognition of a target DNA, the capture of the NP-Pts onto a solid substrate consisting of magnetic microbeads, and finally the detection in minutes with the naked eye of the colour that is generated upon addition of TMB as the chromogenic substrate.

More specifically, the inventors synthesized NP-Pt nanoparticles of about 20 nm in diameter following the procedure described in M. Moglianetti, E. De Luca, D. Pedone, R. Marotta, T. Catelani, B. Sartori, H. Amenitsch, S.F. Retta, and P.P. Pompa "Platinum nanozymes recover cellular ROS homeostasis in oxidative stress-mediated disease model" Nanoscale 8, 3739 (2016) and functionalized the NP-Pt nanoparticles with a polyT probe, following the procedure described for gold nanoparticles in P. Valentini and P.P. Pompa "A Universal Polymerase Chain Reaction Developer" Angewandte Chemie 55, 2157 (2016). Functionalized nanoparticles typically show a greater hydrodynamic radius compared to non-functionalized nanoparticles, as can be verified by DLS analysis. Moreover, nanoparticles functionalized with oligonucleotide probes maintain a high catalytic efficiency compared to non-functionalized nanoparticles (a loss of 20-30% of the colorimetric signal is observed at most), thus guaranteeing high sensitivity of the method provided herein.

The inventors also designed model oligonucleotide probes capable of detecting *E. coli.* The first P1 oligonucleotide probe is biotinylated, the second P2 probe has a polyA tail complementary to the polyT tail of the universal oligonucleotide probe (P0), the target is a single-stranded synthetic sequence used as a template of *E. coli* genomic DNA.

### MODEL SEQUENCES

P1 = 5'-CCTTCCACACCTTCCAAACACACACAAA-iSp18-biotin-3' (SEQ ID NO:1)
P2 = 5'-AAAAAAAAAAAAAAAAAAAAAAAAAAAAAATTTACCACTCAAC-3' (SEQ ID NO:2)
TARGET (T) = 5'-AAGGTGTGGAAGGGTTGAGTGGTAAA-3' (SEQ ID NO:3)

In this way, the P1 probe was captured onto the streptavidin-coupled magnetic microparticles (commercially available, for example, from Invitrogen), while the Poly-A sequence of the P2 probe captured the NP-Pts functionalized with the polyT probe.

In the presence of the target nucleic acid, a sandwich structure was thus formed, in which the NP-Pts are bound to the solid substrate by hybridization of the two oligonucleotide probes P1 and P2 to the target nucleic acid and interaction of the streptavidin on the solid substrate with the biotin on the P1 probe. In the absence of the target, instead, the NP-Pts remain free in solution and are thus washed away.

After a washing step to remove all that was not bound to the solid substrate, the TMB chromogenic peroxidase substrate was added to the reaction mixture, as described in M. Moglianetti et al. 2016. After 5 minutes, a simple inspection with the naked eye made it possible to verify colour appearance and then determine the presence of the target, which was present in the sample in a picomolar or sub-picomolar concentration.

As indicated, the assay has general validity and can be applied to any target nucleic acid, simply by designing two specific probes for the target of interest and keeping all the other components constant, similarly to what occurs in a standard PCR.

### Protocol:

1. 5 µL of magnetic beads are washed twice with a hybridization buffer (B) and resuspended in 5 µL of B;
2. 5 µL of P1 (600 nM) and 5 µL of P2 (100 nM) are incubated with 5 µL of target sequence (target, (T)) at the concentration to be detected for 10 minutes at room temperature;
3. the washed and resuspended magnetic beads are added to the P1, P2 and T mix and incubated for 20 minutes at room temperature, stirring slowly; after the incubation time, the beads are washed twice with B and resuspended in 20 µL of B;
4. 5 µL of 100 pM functionalized platinum nanoparticles are added and allowed to incubate for 15 minutes at room temperature; after the incubation time, the particles are washed three times with B and resuspended in 5 µL of milliQ water;
5. TMB, H₂O₂ and acetate buffer are added and allowed to incubate for 5-10 minutes; observe the colour change. In the presence of the target sequence, even at low concentrations, the solution will become clearly blue and will be detectable with the naked eye. In the absence of the specific target sequence, the solution will remain transparent.

The same experiment was performed with 10-nm palladium NPs functionalized with a polyT probe, similarly to the platinum NPs, obtaining the same colorimetric results as described above, upon capture of the target sequence.

To determine the limit of detection (sensitivity) of the method of the invention, the inventors used a single-stranded synthetic sequence, purchased from IDT, Integrated DNA Technologies, Inc., USA. The stock solution of the target sequence, according to the supplier's indications, had a 100 micromolar concentration. From this solution a series of stepwise dilutions (1:10) was carried out in water, until the concentration became 1 femtomolar. All the dilutions were then tested with the colorimetric method of the invention according to the protocol described above, determining a LOD of 100 femtomolar. At this concentration, after 5 minutes a clear blue colour was observed with the naked eye, whereas the control sample (identical reagents, without the target sequence) remained transparent.

## Claims

1. A kit for the colorimetric detection of a target nucleic acid in a sample, the kit comprising:
- metal nanoparticles (NP) having peroxidase-like catalytic activity, said nanoparticles being functionalized with a universal oligonucleotide probe (P0);
- a first oligonucleotide probe (P1) comprising:
- a 5' target binding sequence (5'TBS) capable of hybridizing to a 5' region of the target nucleic acid, and a 3' sequence which is not capable of hybridizing to the target nucleic acid and which is labelled at its 3' end with an affinity moiety;
- a second oligonucleotide probe (P2) comprising:
- a 3' target binding sequence (3'TBS) capable of hybridizing to a 3' region of the target nucleic acid, and a 5' sequence which is not capable of hybridizing to the target nucleic acid but is capable of hybridizing to the universal oligonucleotide probe (P0) with which said metal nanoparticles (NP) are functionalized; and
- a solid substrate functionalized with the binding partner of said affinity moiety; and
- a chromogenic peroxidase substrate.

2. The kit according to claim 1, wherein the metal nanoparticle (NP) diameter is comprised between 1 nm and 200 nm.

3. The kit according to claim 1 or 2, wherein said metal nanoparticles (NP) are platinum and/or palladium nanoparticles.

4. The kit according to any one of claims 1 to 3, wherein said universal oligonucleotide probe (P0) consists of or comprises a polyT tail and wherein the 5' sequence of said second oligonucleotide probe (P2) consists of or comprises a polyA tail, or vice versa.

5. The kit according to any one of claims 1 to 4, wherein said affinity moiety is biotin and said affinity moiety binding partner is avidin or streptavidin.

6. The kit according to any one of claims 1 to 5, wherein said chromogenic peroxidase substrate is 3,3',5,5'-tetramethylbenzidine (TMB).

7. The kit according to any one of claims 1 to 6, wherein said solid substrate is made of magnetic beads.

8. A method of colorimetric detection of a target nucleic acid in a sample, comprising the steps of:
(i) optionally subjecting the sample to thermal denaturation; and subsequently
(ii) contacting the sample with the following reagents:
- metal nanoparticles (NP) having peroxidase-like catalytic activity, said nanoparticles being functionalized with a universal oligonucleotide probe (P0);
- a first oligonucleotide probe (P1) comprising:
- a 5' target binding sequence (5'TBS) capable of hybridizing to a 5' region of the target nucleic acid, and a 3' sequence which is not capable of hybridizing to the target nucleic acid and is labelled at its 3' end with an affinity moiety;
- a second oligonucleotide probe (P2) comprising:
- a 3' target binding sequence (3'TBS) capable of hybridizing to a 3' region of the target nucleic acid, and a 5' sequence which is not capable of hybridizing to the target nucleic acid but is capable of hybridizing to the universal oligonucleotide probe (P0) with which said metal nanoparticles (NP) are functionalized, and
- a solid substrate functionalized with the binding partner of said affinity moiety;
(iii) separating the sample material bound to the solid substrate from the sample material not bound to the solid substrate; and
(iv) reacting the sample material bound to the solid substrate with a chromogenic peroxidase substrate capable of generating a colour upon oxidation by the metal nanoparticles (NP) having a peroxidase-like catalytic activity, the appearance of the colour being indicative of the presence of the target nucleic acid in the sample.

9. The method according to claim 8, wherein the target nucleic acid is double stranded DNA (dsDNA).

10. The method according to claim 8 or 9, wherein said metal nanoparticles (NP) are as defined in claim 2 and/or 3.

11. The method according to any one of claims 8 to 10, wherein said universal oligonucleotide probe (P0) consists of or comprises a polyT tail and wherein the 5' sequence of said second oligonucleotide probe (P2) consists of or comprises a polyA tail, or vice versa.

12. The method according to any one of claims 8 to 11, wherein the chromogenic peroxidase substrate is 3,3',5,5'-tetramethylbenzidine (TMB).

13. The method according to any one of claims 8 to 12, wherein said affinity moiety is biotin and said affinity moiety binding partner is avidin or streptavidin.

14. The method according to any one of claims 8 to 13, wherein said solid substrate is made of magnetic beads.

## Patentansprüche

1. Kit zum kolorimetrischen Nachweis einer Ziel-Nukleinsäure in einer Probe, wobei das Kit umfasst:
- Metall-Nanopartikel (NP) mit Peroxidase-artiger katalytischer Aktivität, wobei die Nanopartikel mit einer universellen Oligonukleotid-Sonde (P0) funktionalisiert sind;
- eine erste Oligonukleotid-Sonde (P1), umfassend:
eine 5'-Zielbindungssequenz (5'TBS), die in der Lage ist, mit einer 5'-Region der Ziel-Nukleinsäure zu hybridisieren, und eine 3'-Sequenz, die nicht in der Lage ist, an die Ziel-Nukleinsäure zu hybridisieren, und die an ihrem 3'-Ende mit einer Affinitätskomponente markiert ist;
- eine zweite Oligonukleotid-Sonde (P2), umfassend:
eine 3'-Zielbindungssequenz (3'TBS), die in der Lage ist, an eine 3'-Region der Ziel-Nukleinsäure zu hybridisieren, und eine 5'-Sequenz, die nicht in der Lage ist, an die Ziel-Nukleinsäure zu hybridisieren, aber in der Lage ist, an die universelle Oligonukleotid-Sonde (P0) zu hybridisieren, mit der die Metall-Nanopartikel (NP) funktionalisiert sind; und
- ein festes Substrat, das mit dem Bindungspartner der Affinitätskomponente funktionalisiert ist; und
- ein chromogenes Peroxidase-Substrat.

2. Kit nach Anspruch 1, wobei sich der Durchmesser der Metall-Nanopartikel (NP) zwischen 1 nm und 200 nm bewegt.

3. Kit nach Anspruch 1 oder 2, wobei die Metall-Nanopartikel (NP) Platin- und/oder Palladium-Nanopartikel sind.

4. Kit nach einem der Ansprüche 1 bis 3, wobei die universelle Oligonukleotid-Sonde (P0) aus einem polyT-Schwanz besteht oder diesen umfasst und wobei die 5'-Sequenz der zweiten Oligonukleotid-Sonde (P2) aus einem polyA-Schwanz besteht oder diesen umfasst, oder umgekehrt.

5. Kit nach einem der Ansprüche 1 bis 4, wobei die Affinitätskomponente Biotin ist und der Bindungspartner der Affinitätskomponente Avidin oder Streptavidin ist.

6. Kit nach einem der Ansprüche 1 bis 5, wobei das chromogene Peroxidase-Substrat 3,3 ,5,5'-Tetramethylbenzidin (TMB) ist.

7. Kit nach einem der Ansprüche 1 bis 6, wobei das feste Substrat aus magnetischen Kügelchen besteht.

8. Verfahren zum kolorimetrischen Nachweis einer Ziel-Nukleinsäure in einer Probe, mit den folgenden Schritten:
(i) optionales Unterziehen der Probe einer thermischen Denaturierung; und anschließend
(ii) Inkontaktbringen der Probe mit den folgenden Reagenzien:
Metall-Nanopartikeln (NP) mit Peroxidase-artiger katalytischer Aktivität, wobei die Nanopartikel mit einer universellen Oligonukleotid-Sonde (P0) funktionalisiert sind;
- wobei eine erste Oligonukleotid-Sonde (P1) umfasst:
eine 5'-Zielbindungssequenz (5'TBS), die in der Lage ist, an eine 5'-Region der Ziel-Nukleinsäure zu hybridisieren, und eine 3'-Sequenz, die nicht in der Lage ist, an die Ziel-Nukleinsäure zu hybridisieren, und die an ihrem 3'-Ende mit einer Affinitätskomponente markiert ist;
- wobei eine zweite Oligonukleotid-Sonde (P2) umfasst:
eine 3'-Zielbindungssequenz (3 TBS), die in der Lage ist, an eine 3'-Region der Ziel-Nukleinsäure zu hybridisieren, und eine 5'-Sequenz, die nicht in der Lage ist, an die Ziel-Nukleinsäure zu hybridisieren, aber in der Lage ist, an die universelle Oligonukleotid-Sonde (P0) zu hybridisieren, mit der die Metall-Nanopartikel (NP) funktionalisiert sind, und
- wobei ein festes Substrat mit dem Bindungspartner der Affinitätskomponente funktionalisiert ist;
(iii) Trennen des an das feste Substrat gebundenen Probenmaterials von dem nicht an das feste Substrat gebundenen Probenmaterial; und
(iv) Umsetzen des an das feste Substrat gebundenen Probenmaterials mit einem chromogenen Peroxidase-Substrat, das in der Lage ist, bei Oxidation durch die Metall-Nanopartikel (NP), die eine Peroxidase-artige katalytische Aktivität aufweisen, eine Farbe zu erzeugen, wobei das Erscheinen der Farbe das Vorhandensein der Ziel-Nukleinsäure in der Probe anzeigt.

9. Verfahren nach Anspruch 8, wobei die Ziel-Nukleinsäure doppelsträngige DNA (dsDNA) ist.

10. Verfahren nach Anspruch 8 oder 9, wobei die Metall-Nanopartikel (NP) wie in Anspruch 2 und/oder 3 definiert sind.

11. Verfahren nach einem der Ansprüche 8 bis 10, wobei die universelle Oligonukleotid-Sonde (P0) aus einem polyT-Schwanz besteht oder diesen umfasst und wobei die 5'-Sequenz der zweiten Oligonukleotid-Sonde (P2) aus einem polyA-Schwanz besteht oder diesen umfasst, oder umgekehrt.

12. Verfahren nach einem der Ansprüche 8 bis 11, wobei das chromogene Peroxidase-Substrat 3,3 ,5,5'-Tetramethylbenzidin (TMB) ist.

13. Verfahren nach einem der Ansprüche 8 bis 12, wobei die Affinitätskomponente Biotin ist und der Bindungspartner der Affinitätskomponente Avidin oder Streptavidin ist.

14. Verfahren nach einem der Ansprüche 8 bis 13, wobei das feste Substrat aus magnetischen Kügelchen besteht.

## Revendications

1. Kit de détection colorimétrique d'un acide nucléique cible dans un échantillon, le kit comprenant :
- des nanoparticules métalliques (NP) présentant une activité catalytique peroxydasique, lesdites nanoparticules étant fonctionnalisées avec une sonde oligonucléotidique universelle (P0) ;
- une première sonde oligonucléotidique (P1) comprenant :
- une séquence de liaison à la cible 5' (5'TBS) capable de s'hybrider à une région 5' de l'acide nucléique cible, et une séquence 3' qui n'est pas capable de s'hybrider à l'acide nucléique cible et qui est marquée à son extrémité 3' avec une fraction d'affinité ;
- une deuxième sonde oligonucléotidique (P2) comprenant :
- une séquence de liaison à la cible 3' (3'TBS) capable de s'hybrider à une région 3' de l'acide nucléique cible, et une séquence 5' qui n'est pas capable de s'hybrider à l'acide nucléique cible mais qui est capable de s'hybrider à la sonde oligonucléotidique universelle (P0) avec laquelle lesdites nanoparticules métalliques (NP) sont fonctionnalisées ; et
- un substrat solide fonctionnalisé avec le partenaire de liaison de ladite fraction d'affinité ; et
- un substrat de peroxydase chromogène.

2. Kit selon la revendication 1, dans lequel le diamètre de la nanoparticule métallique (NP) est compris entre 1 nm et 200 nm.

3. Kit selon la revendication 1 ou 2, dans lequel lesdites nanoparticules métalliques (NP) sont des nanoparticules de platine et/ou de palladium.

4. Kit selon l'une quelconque des revendications 1 à 3, dans lequel ladite sonde oligonucléotidique universelle (P0) se compose de ou comprend une queue polyT, et dans lequel la séquence 5' de ladite deuxième sonde oligonucléotidique (P2) se compose de ou comprend une queue polyA, ou vice-versa.

5. Kit selon l'une quelconque des revendications 1 à 4, dans lequel ladite fraction d'affinité est une biotine et ledit partenaire de liaison de la fraction d'affinité est une avidine ou une streptavidine.

6. Kit selon l'une quelconque des revendications 1 à 5, dans lequel ledit substrat de peroxydase chromogène est la 3,3',5,5'-tétraméthylbenzidine (TMB).

7. Kit selon l'une quelconque des revendications 1 à 6, dans lequel ledit substrat solide est constitué de billes magnétiques.

8. Procédé de détection colorimétrique d'un acide nucléique cible dans un échantillon, comprenant les étapes consistant à :
(i) soumettre en option l'échantillon à une dénaturation thermique ; puis
(ii) mettre l'échantillon en contact avec les réactifs suivants :
- des nanoparticules métalliques (NP) présentant une activité catalytique peroxydasique, lesdites nanoparticules étant fonctionnalisées avec une sonde oligonucléotidique universelle (P0) ;
- une première sonde oligonucléotidique (P1) comprenant :
- une séquence de liaison à la cible 5' (5'TBS) capable de s'hybrider à une région 5' de l'acide nucléique cible, et une séquence 3' qui n'est pas capable de s'hybrider à l'acide nucléique cible et qui est marquée à son extrémité 3' avec une fraction d'affinité ;
- une deuxième sonde oligonucléotidique (P2) comprenant :
- une séquence de liaison à la cible 3' (3'TBS) capable de s'hybrider à une région 3' de l'acide nucléique cible, et une séquence 5' qui n'est pas capable de s'hybrider à l'acide nucléique cible mais qui est capable de s'hybrider à la sonde oligonucléotidique universelle (P0) avec laquelle lesdites nanoparticules métalliques (NP) sont fonctionnalisées, et
- un substrat solide fonctionnalisé avec le partenaire de liaison de ladite fraction d'affinité ;
(iii) séparer le matériau d'échantillon lié au substrat solide du matériau d'échantillon non lié au substrat solide ; et
(iv) faire réagir le matériau d'échantillon lié au substrat solide avec un substrat de peroxydase chromogène capable de générer une couleur en cas d'oxydation par les nanoparticules métalliques (NP) ayant une activité catalytique peroxydasique, l'apparence de la couleur étant indicatrice de la présence de l'acide nucléique cible dans l'échantillon.

9. Procédé selon la revendication 8, dans lequel l'acide nucléique cible est un ADN double brin (ADNdb).

10. Procédé selon la revendication 8 ou 9, dans lequel lesdites nanoparticules métalliques (NP) sont telles que définies dans les revendications 2 et/ou 3.

11. Procédé selon l'une quelconque des revendications 8 à 10, dans lequel ladite sonde oligonucléotidique universelle (P0) se compose de ou comprend une queue polyT, et dans lequel la séquence 5' de ladite deuxième sonde oligonucléotidique (P2) se compose de ou comprend une queue polyA, ou vice-versa.

12. Procédé selon l'une quelconque des revendications 8 à 11, dans lequel le substrat de peroxydase chromogène est la 3,3',5,5'-tétraméthylbenzidine (TMB).

13. Procédé selon l'une quelconque des revendications 8 à 12, dans lequel ladite fraction d'affinité est une biotine et ledit partenaire de liaison de la fraction d'affinité est une avidine ou une streptavidine.

14. Procédé selon l'une quelconque des revendications 8 à 13, dans lequel ledit substrat solide est constitué de billes magnétiques.
